# EUROPÄISCHE PATENTSCHRIFT

(11) **EP 1 044 648 B1**
(45) Veröffentlichungstag und Bekanntmachung des Hinweises auf die Patenterteilung: **26.11.2003**
(21) Anmeldenummer: 00105430.3
(22) Anmeldetag: 15.03.2000
(51) Int. Cl.: A61B 5/107

(54) **Verfahren zum Vermessen eines Körperbereichs**
Method for measuring part of body
Procédé pour mesurer une partie du corps

(30) Priorität: 15.04.1999 DE 19916978
(43) Veröffentlichungstag der Anmeldung: 18.10.2000
(73) Patentinhaber: Otto Bock HealthCare GmbH, 37115 Duderstadt (DE)
(72) Erfinder: Pusch, Martin, 37115 Duderstadt (DE); Reynolds, Dave, Middlesex TW11 8AP (GB)
(74) Vertreter: Bremer, Ulrich, Dipl.-Phys.

(56) Entgegenhaltungen:
- DE-A- 4 417 872
- FR-A- 2 748 590
- US-A- 1 937 433
- US-A- 5 706 419

## Beschreibung

Die Erfindung bezieht sich auf ein Verfahren zum Vermessen eines Körperbereichs eines menschlichen Körpers.

Derartige Verfahren werden insbesondere für die Anfertigung von Prothesen, z.B. für Gliedmaßenstümpfe, sowie Orthesen bzw. medizinische Stützelemente, wie z.B. Kompressionsstrümpfe, benötigt, um eine individuelle Anpassung der Prothese bzw. Orthese an den jeweiligen Körperbereich zu ermöglichen.

Die Vermessung des jeweiligen Körperbereichs erfordert zum einen eine hohe Genauigkeit, damit die anschließend auf Grundlage der Vermessungsdaten angefertigte Prothese oder Orthese ein hohen Tragekomfort bietet. Zum anderen sollte die Vermessung des Körperbereichs in hinreichend kurzer Zeit und mit einem relativ geringen apparativen Aufwand möglich sein, damit der Patient vor Ort in relativ kurzer Zeit vermessen werden kann und ihm somit eine Fahrt zu einer speziellen, evtl. weiter entfernt vorhandenen Meßapparatur sowie eine längere zeitliche Beanspruchung durch den Meßvorgang erspart bleiben.

Es ist hierzu bekannt, den Körperbereich z.B. durch Maßbänder zu vermessen. Hierbei müssen z.B. bei einem Gliedmaßenstumpf neben der Länge im allgemeinen mehrere Umfangsmessungen durchgeführt werden, die dennoch im allgemeinen keine allzu große Genauigkeit bei der Vermessung erlauben. Weiterhin ist es bekannt, von den betreffenden Körperbereichen Abdrücke zu nehmen. Ein derartiges Verfahren ist jedoch aufwendig und langwierig, weiterhin muss ein Abdruck des Körperbereichs zur Herstellung der Prothese bzw. Orthese gegebenenfalls zunächst zu dem jeweiligen Hersteller befördert werden, wodurch neben den Transportkosten auch eine größere Zeitverzögerung auftreten kann.

Ein aus der Druckschrift US-A-1 937 433 bekanntes Verfahren bestimmt durch Vergleich zumindest zweier unter verschieden Winkeln aufgenommen Abbildungen eines zu vermessenden Körpers vor einer bemaßten Referenztafel relative Abstände auf diesem Körper.

Der Erfindung liegt die Aufgabe zugrunde, gegenüber dem Stand der Technik Verbesserungen zu schaffen und insbesondere ein Verfahren zur Verfügung zu stellen, das eine genaue Vermessung des Körperbereichs mit relativ wenig Aufwand und in relativ kurzer Zeit ermöglicht.

Diese Aufgabe wird gelöst, indem ein Referenzgegenstand mit einer Referenzstrecke vorbekannter Länge und einer Winkeleinstelleinrichtung in festem räumlichen Bezug zu dem Körperbereich, z.B. an dem Körperbereich, angeordnet wird, eine Kamera mittels der Winkeleinstelleinrichtung in einer zur Referenzstrecke senkrechten ersten Betrachtungsrichtung in einer ersten Kameraposition angeordnet wird, in der sie den Körperbereich mit dem Referenzgegenstand erfaßt, und ein erstes Bild aufgenommen wird, eine Kamera mittels der Winkeleinstelleinrichtung in einer zur Referenzstrecke senkrechten zweiten Betrachtungsrichtung in einer zweiten Kameraposition angeordnet wird, in der sie den Körperbereich mit dem Referenzgegenstand erfaßt, und ein zweites Bild aufgenommen wird, aus der Länge der Referenzstrecke und den Abbildungen der Referenzstrecke auf den Bildern die Abstände des Referenzgegenstandes von den Kamerapositionen als erster Referenzabstand und zweiter Referenzabstand ermittelt werden, für jedes Bild eine im dreidimensionalen Raum verlaufende Konturlinie aus dem Referenzabstand und der Abbildung der Konturlinie auf Grundlage eines vorgespeicherten Referenzmodells bestimmt wird, und ein individuelles Modell des Körperbereichs aus den Konturlinien und dem Referenzmodell ermittelt wird.

Erfindungsgemäß wird somit ein Referenzmodell für den betreffenden Körperbereich verwendet. Die Verwendung eines derartigen Referenzmodells ist bei Orthesen, die einen Körperbereich stützen, im allgemeinen mit relativ hoher Genauigkeit möglich, da das Referenzmodell einen Bereich eines zumindest weitgehend intakten Körpers nachbildet. Ein deratiges Referenzmodell ist auch bei der Anpassung von Prothesen, bei denen ein fehlendes Körperteil ersetzt wird, sinnvoll, da der betreffende Körperbereich, z.B. ein Gliedmaßenstumpf, im allgemeinen operationstechnisch auf bekannte Weise vorbehandelt wird. Hierzu werden insbesondere die Knochen und Muskeln an den jeweiligen Körperstellen auf weitgehend bekannte Weise freigelegt.

Da erfindungsgemäß lediglich ein Referenzgegenstand mit festem räumlichen Bezug zu dem betreffenden Körperbereich, insbesondere an dem betreffenden Körperbereich, angebracht, und mindestens zwei Bilder aus verschiedenen Kamerapositionen von dem betreffenden Körperbereich aufgenommen werden müssen, kann der Körperbereich relativ schnell vermessen werden, wobei lediglich der Einsatz des Referenzgegenstandes und einer Kamera notwendig ist. Die Bilder können anschließend an den Hersteller der Prothesen bzw. Orthesen geschickt, gegebenenfalls auch gefaxt werden. Die Bestimmung der Konturlinien aus den Bildern und die Berechnung eines individuellen Modells des Körperbereichs durch Bestimmung der Referenzabstände sowie auf Grundlage des Referenzmodells kann dabei bei dem Hersteller erfolgen, so dass beispielsweise ein aufwendiger Transport von Abdrücken oder Abgüssen des Körperbereichs überflüssig wird.

Erfindungsgemäß weist der Referenzgegenstand sowohl eine Referenzstrecke vorbekannter Länge als auch eine z.B. visuelle Winkeleinstelleinrichtung zur Einstellung der Betrachtungsrichtung, vorzugsweise der optischen Achse der Kamera auf. Somit kann die Kamera mit Hilfe der Winkeleinstelleinrichtung relativ einfach in einer zur Referenzstrecke senkrechten Richtung positioniert werden, ohne dass der zu vermessende Körperbereich in Halterungen oder Gestelle positioniert oder eingespannt werden muss. Die Konturlinien des Körperbereichs können aus einer einfachen nachträglichen Vermessung anhand der aufgenommenen Bilder bestimmt werden, ohne dass eine direkte Vermessung oder Abtastung des Körperbereichs vor Ort notwendig ist. Somit kann ein individuelles Modell durch rechnerische Ermittlung bzw. rechnerische Anpassung bestimmt werden. Vorteilhafterweise kann zur Bestimmung der Konturlinien der betreffende Körperbereich hell beleuchtet und vor einem dunklen Hintergrund aufgenommen werden. Insbesondere kann ein kontrastverbessernder, z.B. heller Überzug über den Körperbereich gelegt oder gestreift werden, an dem auch gegebenenfalls Bereiche oder Stellen markiert werden können.

Da erfindungsgemäß die Abstände der Kamera von dem Referenzgegenstand aus den Bildern ermittelt werden, ist eine aufwendige Positionierung des Körperbereichs in einem festen, vorgegebenen Abstand zu der Kamera überflüssig, der eine Ruhigstellung des Patienten erfordern würde und im allgemeinen dennoch zu einer höheren Ungenauigkeit führen würde.

Der Referenzgegenstand kann vorteilhafterweise direkt an dem Körperbereich befestigt, z.B. geklebt werden, sodass ein eindeutiger räumlicher Bezug des Referenzgegenstands zu dem Körperbereich gegeben ist. Hierdurch kann der Patient den betreffenden Körperbereich zwischen den Aufnahmen sogar etwas bewegen, da durch den an dem Körperbereich befestigten Referenzgegenstand ein körpereigenes Bezugssystems definiert wird, dass bei Bewegungen des Körperbereichs grundsätzlich nicht verändert wird.

Die Referenzstrecke kann an dem Referenzgegenstand insbesondere durch zwei Markierungen gebildet werden, deren Abstand vorbekannt ist. Die Winkeleinstelleinrichtung kann beispielsweise durch ein oder zwei Vorsprünge gebildet werden, die senkrecht von der Längenskala abstehen und somit bei Ausrichtung in der Betrachtungsrichtung die senkrechte Anordnung der Längenskala zur optischen Achse gewährleisten. Vorteilhafterweise können die Vorsprünge gleichzeitig als Markierungen dienen, die mit bekanntem Abstand zueinander angeordnet sind. Die Vorsprünge können dabei auch an einer Schiene verschiebbar sein, sodass ein Abstand voreingestellt werden kann.

Die Vorsprünge können insbesondere als Rippen, Stege oder Blätter ausgebildet sein, die senkrecht von der Längenskala abstehen. Indem die Seitenflächen bzw. Ober- und Unterseiten des Vorsprungs bzw. der Vorsprünge farbig ausgebildet sind, kann eine einfache Einstellung der senkrechten Betrachtungsrichtung erreicht werden, indem die Kamera derartig positioniert wird, dass die farbigen Seitenflächen nicht mehr oder lediglich in vernachlässigbarem Umfang erkannt werden.

Indem für die verschiedenen Aufnahmen die gleiche Kamera verwendet wird, ist der apparative Aufwand für die Vermessung relativ gering. Weiterhin können sich in diesem Fall nicht die verschiedenen optischen Fehler unterschiedlicher Kameras, z.B. eine Dejustierung der optischen Achse, addieren. Es können jedoch grundsätzlich auch verschiedene Kameras verwendet werden.

Die Bestimmung der tatsächlichen, im dreidimensionalen Raum verlaufenden Konturlinien erfolgt erfindungsgemäß mit Hilfe des Referenzmodels. Vorteilhafterweise wird zunächst eine Referenzebene bestimmt, in der die Referenzstrecke liegt und die senkrecht zu der Betrachtungsrichtung der Kamera verläuft. Der Abstand dieser Ebene von der Kameraposition entspricht somit dem ermittelten Referenzabstand. Die Abbildungen der Konturlinien können vorteilhafterweise zunächst auf diese Referenzebene bezogen werden, wobei die Position der Referenzebene im allgemeinen nicht mit der tatsächlichen Position der den Körperbereich einhüllenden Konturlinien übereinstimmt. Anschließend werden aus den Konturlinienprojektionen und den Referenzabständen zunächst die Konturlininen und hieraus das individuelle Modell bestimmt.

Weiterhin könne z.B. prominente Stelle oder Teilbereiche des Körperbereichs, die in zumindest einer der Abbildungen erkannt werden können, herangezogen werden. Statt derartiger markanter Stellen oder Teilbereiche können entsprechend auch Stellen oder Teilbereiche des Körperbereichs markiert werden, insbesondere durch eine Markierung auf einem Überzug des Körperbereichs. Diese markanten oder markierten Stellen oder Teilbereiche können zusätzlich zu den Konturlinien zur Bestimmung des individuellen Modells herangezogen werden. Somit kann ein individuelles Modell aus Konturlinien und weiteren Stellen oder Teilbereichen des Körperbereichs anhand eines Referenzmodells ermittelt werden.

Das erfindungsgemäße Verfahren kann insbesondere zur Vermessung von Gliedmaßen, insbesondere Gliedmaßenstumpfen wie z.B. einem Unterschenkelstumpf verwendet werden, sodass eine nachfolgende Anpassung einer Prothese ermöglicht wird. Weiterhin können auch Körperbereiche wie z.B. ein Fuß vermessen werden, um eine nachträgliche Anpassung einer Orthese, z.B. von Kompressionsstrümpfen, zu ermöglichen.

Die Erfindung wird im folgenden anhand einer Ausführungsform an einigen Zeichnungen näher erläutert. Es zeigen:
- Figur 1 -: eine Meßanordnung zur erfindungsgemäßen Vermessung eines Unterschenkelstumpfs als Körperbereich;
- Figur 2 -: eine Schnittansicht des Unterschenkelstumps durch die Konturlinien mit eingezeichnetem Referenzmodell;
- Figur 3 -: ein Referenzmodell mit Konturlinien;
- Figur 4 -: ein Diagramm zur Veranschaulichung der Projektion von einer Referenzebene auf ein dreidimensionales Modell;
- Figur 5 -: eine Schnittdarstellung von Figur 4 durch die Kamerapunkte.

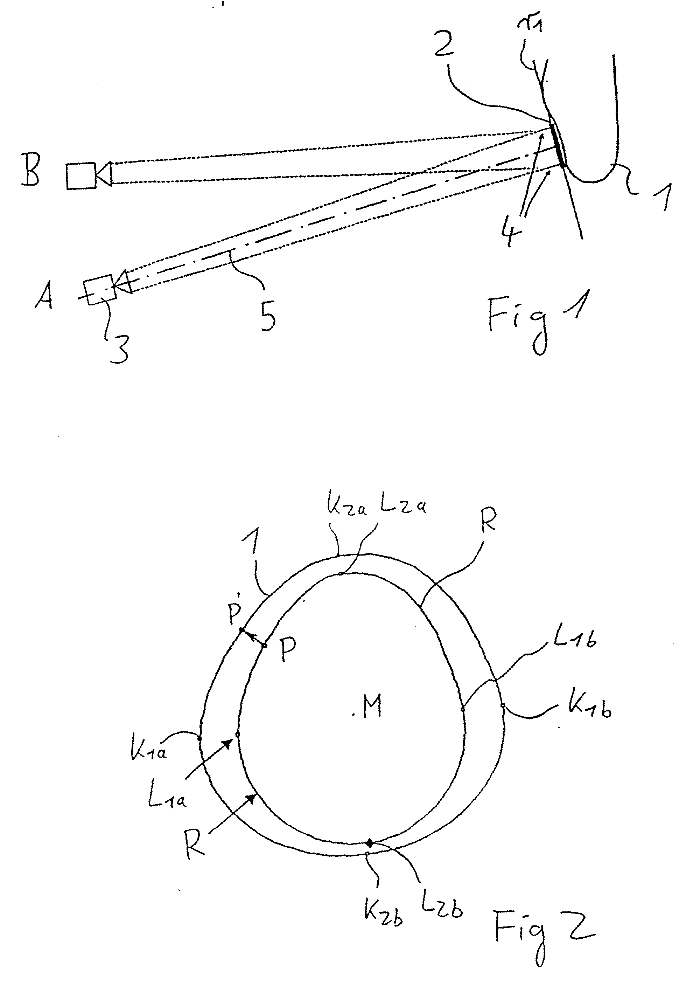

An einem Unterschenkelstumpf 1 als zu vermessendem Körperbereich wird gemäß Figur 1 eine als Referenzgegenstand dienende Referenzlatte 2 befestigt, z.B. angeklebt. Die Ausrichtung der Referenzlatte erfolgt beispielsweie in etwa in Längsrichtung des Unterschenkels. Die Referenzlatte weist an Ihren Enden Stege 4 auf, die beispielsweise auch als Bleche oder Rippen ausgebildet sein können und rechtwinklig von der Referenzlatte abstehen. Die Stege 4 weisen vorteilhafterweise farbige Ober- und Unterseiten auf, wobei beispielsweise die Oberseiten rot und die Unterseiten grün bzw. die äußeren Seiten , d.h. die Oberseite des oberen Stegs und die Unterseite des unteren Stegs, rot, und die inneren Seiten grün gefärbt sein können. Somit können die Stege 4 als Winkeleinstelleinrichtung dienen, um eine zu der Referenzlatte senkrechte Betrachtungsrichtung einzustellen. Somit kann eine Kamera 3 im rechten Winkel zu der Referenzlatte positioniert werden, indem überprüft wird, ob die farbigen Innen- oder Außenseiten der Stege 4 erkannt werden. Demnach ist in Figur 1 die Position B nicht rechtwinklig zu der Referenzlatte, da die Kamera die farbige Oberseite bzw. äußere Seite des oberen Stegs und die farbige Oberseite bzw. Innenseite des unteren Stegs 4 erkennt. Durch entsprechendes Verstellen kann die Kamera 3 in die Position A verstellt werden, in der die Betrachtungsrichtung senkrecht zu der Referenzlatte 2 verläuft. Somit verläuft die optische Achse 5 der Kamera 3 senkrecht zu der Referenzlatte 2 und durch deren Mittelpunkt. Die Referenzlatte weist eine Referenzstrecke mit bekannter Referenzlänge auf, die vorteilhafterweise durch den Abstand der Stege 4 zueinander gebildet wird.

Als Kamera wird vorteilhafterweise eine Digitalkamera, z.B. eine CCD-Kamera, verwendet.

Die Positionierung der Kamera 3 erfolgt in zwei vorteilhafterweise zueinander zumindest im wesentlichen senkrechten Richtungen um den Unterschenkelstumpf 1 herum. Der Unterschenkelstumpf wird vorteilhafterweise beleuchtet und vor einem dunklen Hintergrund aufgenommen, sodass die nachfolgende Bestimmung der Konturlinie des Unterschenkelstumpf als Kontrast auf dem Bild erleichtert wird. Der tatsächliche, im dreidimensionalen Raum liegende Verlauf der Konturlinien ist dabei aus den Abbildungen der Konturlinien in den Bildern noch nicht bekannt. Insbesondere ist dabei nicht bekannt, in welchem Abstand die Konturlinie von der Kamera 3 liegt.

Die Konturlinien können bei hinreichendem Kontrast aus den Bildern durch eine Bilddatenverarbeitung ermittelt werden, bei der beispielsweise Differenzen von nebeneinanderliegenden Pixeln gebildet werden. Derartige Kantenerkennungen sind beispielsweise von Autofocussystemen bekannt.

Da die Referenzlatte 2 senkrecht zur optischen Achse der Kamera 3 verläuft, kann die Referenzlänge direkt zur Skalierung des von der Kamera 3 aufgenommenen Bildes verwendet werden. Eine Skalierung ist somit für die senkrecht zur optischen Achse 5 und durch die Referenzstrecke verlaufende Referenzebene r1 bekannt.

Die Kamera 3 wird anschließend gemäß Figur 5 in eine zweite Position D verstellt, die ebenfalls senkrecht zu der Referenzlatte 2 liegt. Die Justierung erfolgt somit ohne Veränderung der Referenzlatte 2 mit Hilfe der Stege 4. Somit liegt auch die optische Achse der Kamera in der zweiten Meßposition senkrecht zur Referenzlatte 2, nicht jedoch senkrecht zu der Referenzebende r1 der ersten Messung. Durch die Betrachtungsrichtung bzw. optische Achse der Kamera 3 in der zweiten Kameraposition D kann die zweite Referenzebene r2 definiert werden, innerhalb der wiederum die Achse der Referenzlatte 2 liegt. Somit können zwei oder mehr Bilder in verschiedenen (mindestens zwei) Kamerapositionen aufgenommen werden, wobei jeweils Referenzebenen gebildet werden, in denen die Achse der Referenzlatte 2 liegt und die jeweils senkrecht zur optischen Achse der Kamera liegen.

Figur 2 zeigt einen Schnitt durch den Unterschenkelstumpf 1 in einer Ebene, die durch die beiden Kamerapositionen A,D verläuft. Ein Referenzmodell R wird verwendet, um die Außenfläche des Unterschenkelbereichs 1 nachzubilden. Das Referenzmodell kann dabei für den jeweils betrachteten Körperbereich individuell gestaltet werden, sodass die grobe Formgebung des jeweiligen Körperbereichs, z.B. des Unterschenkelstumpfs 1, bereits in etwa durch das Referenzmodell R wiedergegeben wird. Erfindungsgemäß wird das Referenzmodell R nachfolgend anhand der von der Kamera aufgenommenen Bilder an den tatsächlichen Verlauf der Außenfläche des Unterschenkelstumpfs 1 angepaßt. Hierzu müssen erfindungsgemäß aus den Abbildungen der Konturlinien in den Bildern die tatsächlichen Konturlinien K1 und K2 der Außenfläche des Unterschenkelbereichs 1 bestimmt werden und das Referenzmodell an die Konturlinien K1 und K2 angepaßt werden. Problematisch ist dabei, dass aus den Abbildungen der Konturlinien der tatsächliche Verlauf der Konturlinien K1 und K2 im dreidimensionalen Raum noch nicht bekannt ist und mit Hilfe des Referenzmodells R zunächst bestimmt werden muss. Somit dient das Referenzmodell R sowohl zum Bestimmen der Konturlinien K1 und K2 als auch zum Nachbilden eines individuellen Modells des Unterschenkelstumpfs 1 anhand der Konturlinien K1 und K2.

Da die Kameraposition A,D vorteilhafterweise im wesentlichen zueinander um 90° in der in Figur 5 gezeigten Ebene versetzt sind, liegen die optischen Achsen 5a,5b der Kamera im wesentlichen in etwa in den Referenzebenen R2, R1 der jeweils anderen Position. Eine derartige Anordnung ist jedoch nicht notwendig, da die Kamerapositionen I, II nicht um genau 90° versetzt zu einander sein müssen; grundsätzlich ist es lediglich notwendig, zwei Bilder im zueinander unterschiedlichen Kamerapositionen aufzunehmen.

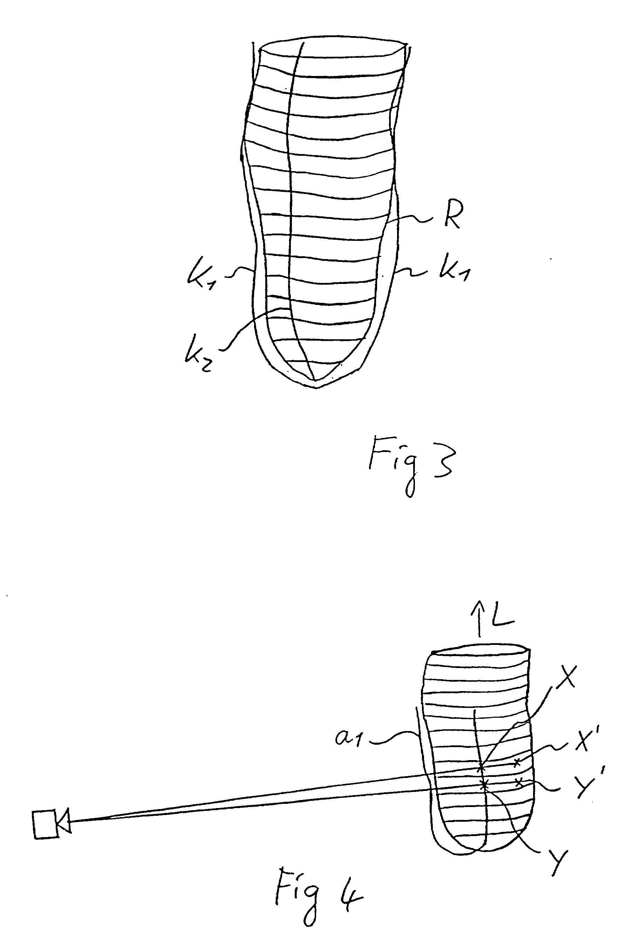

Figur 3 zeigt das erfindungsgemäße Prinzip, ein Referenzmodel R zu verwenden, das an Konturlinien K1, K2 angepaßt werden muss.

Erfindungsgemäß werden vorteilhafterweise gemäß Figur 5 die Abbildungen der Konturenpunkte K1a, K1b, K2a, K2b zunächst auf die jeweiligen Referenzebenden r1, r2 bezogen, wie das in Figur 5 für beide Kamerapositionen gezeigt ist. Es werden demnach Konturlinienprojektionen a1a,a1b,a2a,a2b in den jeweiligen Referenzebenen r1, r2 gebildet. In diesen Referenzebenen ist die Skalierung durch die Referenzlänge der Referenzstrecke bekannt. Nachfolgend müssen die einzelnen Punkte a1a,a1b,a2a,a2b der Konturlinienprojektionen den Konturlinienpunkten K1a,K1b,K2a,K2b des Unterschenkelbereichs angepaßt werden.

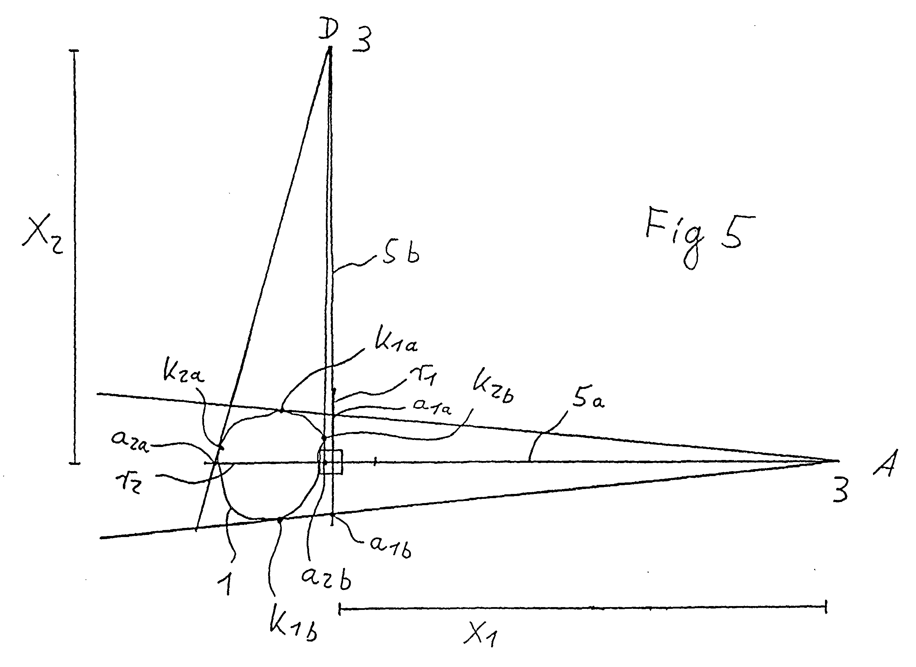

In Figur 5 ist dabei zunächst ein Schnitt durch den Unterschenkelstumpf 1 in der Ebene gezeigt, der durch die optischen Achsen 5a, 5b gebildet wird. Für die Berechnung der Konturlinienpunkte in den weiteren Ebenen kann anschließend analog verfahren werden, wobei die Kamerapunkte A,D jeweils in diesen Ebenen liegen. Anschließend kann die Längserstreckung des Unterschenkelstupfs in Richtung L in Figur 4 an die Skalierung der Referenzlänge angepasst werden.

## Patentansprüche

1. Verfahren zum Vermessen eines Körperbereichs (1) eines menschlichen Körpers, bei dem
ein Referenzgegenstand (2) mit einer Referenzstrecke vorbekannter Länge und einer Winkeleinstelleinrichtung (4) in festem räumlichen Bezug zu dem Körperbereich (1) angeordnet wird,
eine Kamera (3) mittels der Winkeleinstelleinrichtung (4) in einer zur Referenzstrecke senkrechten ersten Betrachtungsrichtung in einer ersten Kameraposition (A) angeordnet wird, in der sie den Körperbereich (1) mit dem Referenzgegenstand (2) erfaßt, und ein erstes Bild aufgenommen wird,
eine Kamera (3) mittels der Winkeleinstelleinrichtung (4) in einer zur Referenzstrecke senkrechten zweiten Betrachtungsrichtung in einer zweiten Kameraposition (D) angeordnet wird, in der sie den Körperbereich (1) mit dem Referenzgegenstand (2) erfaßt, und ein zweites Bild aufgenommen wird,
aus der Länge der Referenzstrecke und den Abbildungen der Referenzstrecke auf den Bildern die Abstände des Referenzgegenstandes (2) von den Kamerapositionen als erster Referenzabstand (X1) und zweiter Referenzabstand (X2) ermittelt werden,
für jedes Bild eine im dreidimensionalen Raum verlaufende Konturlinie (K1,K2) aus dem Referenzabstand (X1,X2) und der Abbildung der Konturlinie auf Grundlage eines vorgespeicherten Referenzmodells (R) bestimmt wird, und
ein individuelles Modell (M) des Körperbereichs (1) aus den Konturlinien (K1, K2) und dem Referenzmodell (R) ermittelt wird.

2. Verfahren nach Anspruch 1, **dadurch gekennzeichnet, dass** für jede Kameraposition (A,D) eine Referenzebene (r1, r2) gebildet wird, in der die Referenzstrecke liegt und die senkrecht zu der Betrachtungsrichtung der Kamera verläuft,
für jede Kameraposition (A,D) aus dem Referenzabstand (X1,X2) und der Abbildung der Konturlinie (K1,K2) eine Konturlinienprojektion (al, a2), die einer Projektion der Konturlinie auf die Referenzebene entspricht, ermittelt wird, und
aus den Konturlinienprojektionen (a1, a2), den Referenzabständen (X1,X2) und dem Referenzmodell (R) das individuelle Modell (M) des Körperbereichs (1) gebildet wird.

3. Verfahren nach Anspruch 1 oder 2, **dadurch gekennzeichnet, dass** über dem Körperbereich (1) ein Überzug, insbesondere ein kontrastverbessernder Überzug, angebracht wird.

4. Verfahren nach einem der Ansprüche 1 bis 3, **dadurch gekennzeichnet, dass** eine senkrecht zu den Betrachtungsrichtungen verlaufende Länge des individuellen Modells (M) nachfolgend aus einem Vergleich der Referenzstrecke mit einem Abstand zweier Punkte des Körperbereichs ermittelt wird.

5. Verfahren nach Anspruch 4, **dadurch gekennzeichnet, dass** zumindest einer der Punkte ein Ende des Körperbereichs und/oder eine markierte Stelle, vorzugsweise eine auf einem Überzug markierte Stelle, ist.

6. Verfahren nach einem der Ansprüche 1 bis 5, **dadurch gekennzeichnet, dass** bei der Ermittlung des individuellen Modells (M) des Körperbereichs neben den Konturlinien und dem Referenzmodell weiterhin Abbildungen von Stellen oder Teilbereichen des Körperbereichs (1), die in zumindest einer der Abbildungen des Körperbereichs erkennbar sind, verwendet werden.

7. Verfahren nach einem der Ansprüche 1 bis 6, **dadurch gekennzeichnet, dass** der Referenzgegenstand (2) an dem Körperbereich (1) befestigt, vorzugsweise festgeklebt wird.

8. Verfahren nach einem der Ansprüche 1 bis 7, **dadurch gekennzeichnet, dass** der Referenzgegenstand (2) zwei Markierungen (4) mit bekanntem Abstand aufweist, die die Referenzstrecke festlegen.

9. Verfahren nach einem der Ansprüche 1 bis 8, **dadurch gekennzeichnet, dass** die Winkeleinstelleinrichtung eine visuelle Winkeleinstelleinrichtung ist und mindestens einen senkrecht zu der Referenzstrecke verlaufenden Vorsprung (4) aufweist.

10. Verfahren nach Anspruch 9, **dadurch gekennzeichnet, dass** die Winkeleinstelleinrichtung zwei voneinander beabstandete, senkrecht zur Referenzstrecke verlaufende Vorsprünge (4) aufweist, die zwischen sich die Referenzstrecke ausbilden.

11. Verfahren nach Anspruch 10, **dadurch gekennzeichnet, dass** der Vorsprung (4) bzw. die Vorsprünge (4) farbige Seitenflächen bzw. farbige Ober- und Unterseiten aufweisen.

12. Verfahren nach einem der Ansprüche 1 bis 11, **dadurch gekennzeichnet, dass** in den verschiedenen Kamerapositionen die gleiche Kamera (3) verwendet wird.

13. Verfahren nach einem der Ansprüche 1 bis 12, **dadurch gekennzeichnet, dass** als Körperbereich ein Gliedmaßenstumpf, vorzugsweise ein Unterschenkelstumpf, vermessen wird.

14. Verfahren nach einem der Ansprüche 1 bis 12, **dadurch gekennzeichnet, dass** als Körperbereich eine Gliedmaße, vorzugsweise ein Fuß, vermessen wird.

## Claims

1. Method of measuring a body region (1) of a human body, in which a reference object (2) with a reference zone of a previously known length and an angle-setting device (4) is arranged in a fixed spatial relationship with the body region (1), a camera (3) is arranged by means of the angle-setting device (4) in a first viewing direction, perpendicular to the reference zone, in a first camera position (A), in which it picks up the body region (1) with the reference object (2) and a first image is recorded, a camera (3) is arranged by means of the angle-setting device (4) in a second viewing direction, perpendicular to the reference zone, in a second camera position (D), in which it picks up the body region (1) with the reference object (2), and a second image is recorded, the distances of the reference object (2) from the camera positions are ascertained from the length of the reference zone and the projected images of the reference zone on the images as a first reference distance (X1) and second reference distance (X2), a contour line (K1, K2), extending in three-dimensional space, is determined for each image from the reference distance (X1, X2) and the projected image of the contour line on the basis of a previously stored reference model (R), and an individual model (M) of the body region (1) is ascertained from the contour lines (K1, K2) and the reference model (R).

2. Method according to Claim 1, **characterized in that** a reference plane (r1, r2) in which the reference zone lies and which extends perpendicularly with respect to the viewing direction of the camera is formed for each camera position (A, D), a contour line projection (a1, a2), which corresponds to a projection of the contour line onto the reference plane, is ascertained for each camera position (A, D) from the reference distance (X1, X2) and the projected image of the contour line (K1, K2), and the individual model (M) of the body region (1) is formed from the contour line projections (a1, a2), the reference distances (X1, X2) and the reference model (R).

3. Method according to Claim 1 or 2, **characterized in that** a coating, in particular a contrast-improving coating, is applied over the body region (1).

4. Method according to one of Claims 1 to 3, **characterized in that** a length of the individual model (M) extending perpendicularly with respect to the viewing directions is subsequently ascertained from a comparison of the reference zone with a distance between two points of the body region.

5. Method according to Claim 4, **characterized in that** at least one of the points is one end of the body region and/or a marked location, preferably a location marked on a coating.

6. Method according to one of Claims 1 to 5, **characterized in that** not only the contour lines and the reference model but also projected images of locations or subregions of the body region (1) which can be detected in at least one of the projected images of the body region are used in the ascertainment of the individual model (M) of the body region.

7. Method according to one of Claims 1 to 6, **characterized in that** the reference object (2) is fastened, preferably adhesively secured, on the body region (1).

8. Method according to one of Claims 1 to 7, **characterized in that** the reference object (2) has two markings (4) at a known distance, which fix the reference zone.

9. Method according to one of Claims 1 to 8, **characterized in that** the angle-setting device is a visual angle-setting device and has at least one projection (4) extending perpendicularly with respect to the reference zone.

10. Method according to Claim 9, **characterized in that** the angle-setting device has two projections (4), which are spaced apart from one another, extend perpendicularly with respect to the reference zone and between them form the reference zone.

11. Method according to Claim 10, **characterized in that** the projection (4) or projections (4) have coloured side surfaces or coloured upper sides and undersides.

12. Method according to one of Claims 1 to 11, **characterized in that** the same camera (3) is used in the different camera positions.

13. Method according to one of Claims 1 to 12, **characterized in that** a stump of a limb, preferably a stump of a lower leg, is measured as the body region.

14. Method according to one of Claims 1 to 12, **characterized in that** a limb, preferably a foot, is measured as the body region.

## Revendications

1. Procédé pour mesurer une partie (1) d'un corps humain, dans lequel
on dispose en relation spatiale fixe avec la partie (1) du corps un objet de référence (2), équipé d'un segment de référence de longueur prédéterminée et d'un dispositif de réglage angulaire (4),
on dispose un appareil de prise de vues (3) dans une première position (A) de l'appareil de prise de vues au moyen du dispositif de réglage angulaire (4), suivant une première direction d'observation perpendiculaire au segment de référence, position dans laquelle l'appareil capte la partie du corps (1) avec l'objet de référence (2), et on prend une première vue,
on dispose un appareil de prise de vues (3) dans une seconde position (D) de l'appareil de prise de vues au moyen du dispositif de réglage angulaire (4), suivant une seconde direction d'observation perpendiculaire au segment de référence, position dans laquelle l'appareil capte la partie (1) du corps avec l'objet de référence (2), et on prend une deuxième vue,
à partir de la longueur du segment de référence et à partir des représentations du segment de référence sur les vues, on obtient les distances de l'objet de référence (2) par rapport aux positions de l'appareil de prise de vues en tant que première distance de référence (X1) et deuxième distance de référence (X2),
pour chaque vue, on détermine une ligne de contour (K1, K2) s'étendant dans l'espace tridimensionnel, à partir de la distance de référence (X1, X2) et de la représentation de la ligne de contour sur la base d'un modèle de référence préenregistré (R), et on obtient un modèle individuel (M) de la partie (1) du corps à partir des lignes de contour (K1, K2) et du modèle de référence (R).

2. Procédé selon la revendication 1, **caractérisé en ce que** pour chaque position de l'appareil de prise de vues (A, D), on constitue un plan de référence (r1, r2) dans lequel se trouve le segment de référence et qui s'étend perpendiculairement à la direction d'observation de l'appareil de prise de vues,
pour chaque position de l'appareil de prise de vues (A, D) on obtient à partir de la distance de référence (X1, X2) et de la représentation des lignes de contour (K1, K2) une projection des lignes de contour (al, a2) qui correspond à une projection de la ligne de contour sur le plan de référence, et
on constitue le modèle individuel (M) de la partie (1) du corps à partir des projections des lignes de contour (al, a2), des distances de référence (X1, X2) et du modèle de référence (R) .

3. Procédé selon la revendication 1 ou 2, **caractérisé en ce qu'**on place sur la partie (1) du corps une housse, en particulier une housse améliorant le contraste.

4. Procédé selon l'une des revendications 1 à 3, **caractérisé en ce qu'**on obtient ensuite à partir d'une comparaison du segment de référence avec une distance de deux points de la partie du corps, une longueur du modèle individuel (M) s'étendant perpendiculairement aux directions d'observation.

5. Procédé selon la revendication 4, **caractérisé en ce qu'**au moins un des points est une extrémité de la partie du corps et/ou un emplacement marqué, de préférence un emplacement marqué sur une housse.

6. Procédé selon l'une des revendications 1 à 5, **caractérisé en ce que** lors de l'obtention du modèle individuel (M) de la partie du corps, on utilise, outre les lignes de contour et le modèle de référence, des représentations d'emplacements ou de zones partielles de la partie (1) du corps, qui sont reconnaissables dans au moins une des représentations de la partie du corps.

7. Procédé selon l'une des revendications 1 à 6, **caractérisé en ce que** l'objet de référence (2) est fixé, de préférence collé sur la partie (1) du corps.

8. Procédé selon l'une des revendications 1 à 7, **caractérisé en ce que** l'objet de référence (2) présente deux marquages (4) à distance connue, qui définissent le segment de référence.

9. Procédé selon l'une des revendications 1 à 8, **caractérisé en ce que** le dispositif de réglage angulaire est un dispositif de réglage angulaire visuel et présente au moins une saillie (4) s'étendant perpendiculairement au segment de référence.

10. Procédé selon la revendication 9; **caractérisé en ce que** le dispositif de réglage angulaire présente deux saillies (4) distantes l'une de l'autre s'étendant perpendiculairement au segment de référence, formant le segment de référence entre elles.

11. Procédé selon la revendication 10, **caractérisé en ce que** la ou les saillie(s) (4) présente(nt) des faces latérales colorées ou selon les cas des dessus et des dessous colorés.

12. Procédé selon l'une des revendications 1 à 11, **caractérisé en ce qu'**on utilise le même appareil de prise de vues (3) dans les différentes positions de l'appareil de prise de vues.

13. Procédé selon l'une des revendications 1 à 12, **caractérisé en ce qu'**on mesure comme partie du corps un moignon de membre, de préférence un moignon de jambe inférieure.

14. Procédé selon l'une des revendications 1 à 12, **caractérisé en ce que** comme partie du corps on mesure un membre, de préférence un pied.
